# EUROPEAN PATENT APPLICATION

(11) **EP 2 613 277 A1**
(43) Date of publication of application: **10.07.2013**
(21) Application number: 12150376.7
(22) Date of filing: 06.01.2012
(51) Int. Cl.: G06F 19/16, G01N 33/94

(54) **Method and system for indentifying compounds that bind and preferably activate a target opioid receptor in a pH-dependent manner**

(71) Applicant: MathPharm GmbH, 10825 Berlin (DE)
(72) Inventor: Stein, Christoph, 10825 Berlin (DE); Weber, Marcus, 12045 Berlin (DE); Scharkoi, Olga, 12107 Berlin (DE); Deuflhard, Peter, 14199 Berlin (DE)
(74) Representative: Boeckh, Tobias

(57) **Abstract**

The invention relates to a method and system for identifying candidate compounds that bind and preferably activate a target opioid receptor in a pH-dependent manner.

## Description

The invention relates to a method and system for identifying candidate compounds that bind and preferably activate a target opioid receptor in a pH-dependent manner. The present invention relates also to the influence of an inflamed (acidic) environment on opioid receptor-ligand interactions and provides a method for identifying candidate compounds that bind and activate a target opioid receptor in a pH-dependent manner, such that opioid receptors are exclusively activated in injured or inflamed tissue. Such compounds are intended for selectively modulating peripheral opioid receptors without causing side effects via opioid receptors in physiological (neutral) environments (for example in the gut or central nervous system), thereby identifying effective analgesic compounds that do not exhibit the side effects commonly associated with conventional natural or synthetic opioid analgesics.

### BACKGROUND OF THE INVENTION

The use of currently available analgesics is limited by major adverse side effects. Non-steroidal analgesics cause gastrointestinal ulcers, bleeding and cardiovascular complications, and opioid drugs produce drowsiness, nausea, respiratory depression, constipation, tolerance or addiction. Following painful peripheral tissue injury and inflammation, opioid receptors on peripheral terminals of primary sensory neurons are upregulated, their G-protein coupling, signaling and recycling is enhanced, and their activation results in potent inhibition of neuronal excitability and analgesia. The augmented intracellular signaling suggests conformational alterations of opioid receptors or ligands in the inflamed environment. Systemically applied conventional opioid agonists (e.g. morphine) activate both peripheral and central (brain) opioid receptors. Activation of opioid receptors in physiological (neutral) environments (e.g. brain, gut) leads to severe side effects, leaving a significant need in the field of pain treatment for compounds that work preferably selectively on peripheral receptors in injured (inflamed) tissue.

Inflammation, accompanied by tissue acidosis, is an essential component of a large group of painful syndromes (Stein, C. et al. Brain Res Rev 60, 90-113 (2009)), including arthritis, skin inflammation, inflammatory back pain, headache (certain types of neurogenic migraine), inflammatory lesions of the nervous system (neuropathic pain), cancer pain, disorders of the immune system (HIV/AIDS, multiple sclerosis), traumatic and postoperative pain. Currently available analgesic drugs are limited by unacceptable side effects such as the central actions of opioids (e.g. sedation, respiratory depression, nausea, addiction, tolerance), the intestinal effects of opioids (constipation, ileus), the gastrointestinal and cardiovascular effects of cyclooxygenase (COX) inhibitors (e.g. bleeding, ulcers, thrombo-embolic complications) and the adverse effects of anticonvulsants and antidepressants (e.g. sedation, ataxia, arrhythmias, coronary vasoconstriction). Therefore, there is need of development of new generations and formulations of analgesics which are devoid of these side effects but retain clinical efficacy.

This can be achieved by targeting opioid receptors on peripheral terminals of dorsal root ganglion (DRG) neurons (also called nociceptors or primary sensory neurons) through the local application of exogenous, or the release of endogenous opioids within injured tissue. Moreover, a large proportion (50 - 100 %) of the antinociceptive effects produced by systemically administered opioids can be mediated by such peripheral opioid receptors, and opioid agonists that do not readily enter the central nervous system (CNS) can have the same analgesic efficacy as conventional opioids (Stein, C. et al. Pharmacol Rev 2011, in press), Craft, R.M., et al., J Pharmacol Exp Ther 275, 1535-42 (1995), amogst others). In addition, peripherally acting opioids have been shown to reduce inflammation by modulating proinflammatory mediators, edema, plasma extravasation and other parameters (Stein, C. et al. Curr Pharm Design 2011, in press). However, despite the fact that peripherally acting opioids have been shown to reduce inflammation, leading to potential success in pain relief strategies regarding modulation of peripheral receptors, few methods have been disclosed for effectively targeting selectively peripheral opioid receptors within injured tissue without effects on either the gut or CNS.

Opioid pharmacology commands a huge armamentarium of non-peptidic and peptidic opioid receptor ligands. The most thoroughly studied include the alkaloid morphine, the piperidine fentanyl, and the enkephalin derivative (D-Ala²,N-MePhe⁴,Gly⁵-ol)-enkephalin (DAMGO). All of these bind to the mu-receptor, the most important receptor type for the mediation of analgesic effects in animals and humans (Zöllner, C. and Stein, C. Handbook of Experimental Pharmacology (Vol. 177) Analgesia (2007)). Chemical modification of such compounds has been accomplished manifold and has produced highly selective ligands for each receptor type as well as agonists that do not enter the CNS. While the latter can induce potent antinociception without central side effects, they are still likely to activate opioid receptors in the physiological (neutral) environment within the submucosal tissue of the gut (when applied orally or systemically), which commonly results in the occurrence of constipation or vomiting. Therefore, it is necessary to develop opioid receptor-ligands with a particular focus on specific activity in the environment in damaged (inflamed) tissue.

Fentanyl is one example (among others e.g. morphine, codeine etc.) of a potent synthetic opioid ("narcotic") analgesic with a rapid onset and short duration of action. It is a potent agonist at mu-opioid receptors. Historically it has been used to treat chronic and breakthrough pain and is commonly used before, during and after procedures as a pain reliever as well as an anaesthetic. However, fentanyl (and others, e.g. morphine, codeine etc.) exhibits significant side effects, such as nausea, vomiting, constipation, dry mouth, somnolence, confusion, hypoventilation, apnoea, tolerance and addiction, in addition to abdominal pain, headache, fatigue, weight loss, dizziness, nervousness, hallucination, anxiety, depression, flu-like symptoms, dyspepsia (indigestion), and urinary retention, which renders fentanyl (and others, e.g. morphine, codeine etc.) in many cases unusable, especially when delivered systemically to a subject in pain. There is a need in the art to develop opioid derivatives that do not exhibit the side effects commonly associated with fentanyl (and others, e.g. morphine, codeine etc.). Many of these side effects are thought to be associated with the effect of fentanyl on the CNS.

The mechanisms underlying the peripheral antinociceptive effects of opioids have been investigated in animals and humans (Stein, C., et al., Nat Med 9, 1003-8 (2003); Pharmacol Rev 2011, in press). To this end, Freund's adjuvant (CFA)-induced hind paw inflammation in rodents has been studied, in addition to the peripheral application of small, systemically inactive doses of morphine in patients undergoing surgery or suffering from chronic arthritis. In several controlled clinical studies the inventors and others have shown that intraarticular morphine produces pain relief of similar efficacy to local anesthetics or steroids without systemic or local side effects. Such effects are apparently mediated by opioid receptors localized on peripheral terminals of DRG neurons. The activation of these receptors reduces neuronal excitability, nociceptive impulse propagation and proinflammatory neuropeptide release. In particular, it has been shown that opioid agonists inhibit the TRPV1 ion channel, which is preferentially expressed in DRG neurons and is activated by the pungent compound capsaicin, protons and other stimuli. The inhibition occurs via opioid receptor-coupled G_{i/o} proteins and the cAMP pathway. Furthermore, it has been shown that peripherally mediated opioid antinociception is particularly prominent within inflamed tissue, and that its efficacy increases with the duration of the inflammatory process. Underlying mechanisms include upregulation of synthesis, peripherally directed axonal transport and G-protein coupling of opioid receptors in DRG neurons.

Importantly, pH-values as low as 4-5 have been measured in painful inflammation (Reeh, P.W. & Steen, K.H., Prog Brain Res 113, 143-51 (1996), Woo, Y.C., et al., Anesthesiology 101, 468-75 (2004). This significant change in pH in inflamed tissue provides a potential mechanism according to which inflammation-specific active agents can be developed, which only exhibit an opioid receptor agonistic function in the area of damage, inflammation and/or pain. The present invention is related to the influence of an inflamed (acidic) environment on opioid receptor-ligand interactions and provides ligands that selectively activate opioid receptors in injured (acidic, low pH) tissue.

In contrast to the large body of information on ligands, relatively little is known about the conformation of opioid receptors. No high-resolution crystallized structure of opioid receptors is available to date. Opioid receptors belong to the rhodopsin/class A subfamily of seven-transmembrane G-protein coupled receptors (GPCR). As such, it is possible to extrapolate information from other members of this subfamily such as the rhodopsin and adrenergic receptors. The crystal structures of rhodopsin, its ligand-free form opsin, the G-protein interacting conformation of opsin, an antagonist-bound adenosine receptor and of beta-adrenergic receptors have however been solved. Furthermore, protonation and pH have been shown to play crucial roles in conformational changes and diffusional dynamics of rhodopsin, in the activation of rhodopsin, in the activation of beta-2-adrenergic receptors and on the secondary structure of an opioid receptor fragment. Protonation of ligands also appears to be important for the activation of opioid receptors. Additional data on opioid receptors are available from random mutagenesis and 3D modeling studies. This information provides a basis for computational homology modeling of opioid ligand-receptor interactions. Importantly however, current modeling strategies neither aim at peripheral opioid receptors nor take into account that opioid ligands and/or receptors may change their conformations in injured tissue. The invention is therefore related to modelling of opioid receptors and ligands, and testing the conformational dynamics of opioid receptor-ligand behavior at different pH conditions.

### SUMMARY OF THE INVENTION

In light of the prior art, the technical problem underlying the present invention is to provide a method or a system for identifying candidate compounds that possess effective analgesic function but do not exhibit side effects on either the gut or central nervous system commonly associated with conventional natural or synthetic opioid analgesics.

This problem is solved by the features of the independent claims. Preferred embodiments of the present invention are provided by the dependent claims.

Therefore, an object of the invention is to provide a method or a system for identifying modified compounds that bind and preferably activate a target opioid receptor in a pH-dependent manner, comprising
a) Modification of base compound to produce a modified compound,
b) Determination of the acid dissociation constant (pKa) of said modified compound,
c) Selection of said modified compound for further processing when pKa of said modified compound is less than 7, preferably between 4 and 7, more preferably between 5 and 7, and
d) Testing the binding of the modified compound in, and preferably activation of,
   said target opioid receptor

In a preferred embodiment the method of the present invention is characterized in that step d) is carried out by simulation of binding of the modified compound in a three-dimensional (3D) binding pocket model for said target opioid receptor.

In a preferred embodiment the method of the present invention is characterized in that step d) is carried out with the modified compound and base compound, successively, in any order, whereby binding of the modified compound in the binding pocket model is compared to binding of the base compound in the binding pocket model, whereby desired modified compounds are identified when the binding of the modified compound is the same or similar in comparison to the base compound.

In a preferred embodiment the method of the present invention is characterized in that one or more of the steps a) to d) are simulations carried out by one or more computer programmes, preferably as individual software modules combined to be automatically carried out, on a computing device.

In one embodiment the method of the present invention is characterized in that the identified modified and/or base compound is chemically synthesized.

In one embodiment the method of the present invention is characterized in that the chemically synthesized modified compound and/or base compound is tested for opioid receptor binding, and preferably opioid receptor agonist function, using in vitro and/or in vivo validation methods.

In one embodiment the method of the present invention is characterized in that the base compound is an opioid or opioid derivative or analogue, which exhibits a nitrogen atom that can be protonated or non-protonated.

The modification of a base compound relates to any modification, preferably changes in the molecular and/or atomic structure. Methods for modification of chemical compounds are known to those in the art, especially to those skilled in chemistry and/or chemical synthesis of pharmaceutical compounds.

In one embodiment the method of the present invention is characterized in that protonation of said nitrogen atom (that can be protonated or non-protonated) occurs in modified compounds with a pKa of less than 7 when at pH values less than 7.

In one embodiment the method of the present invention is characterized in that the target opioid receptor is selected from the group consisting of the mu-receptor, delta-receptor and/or kappa-receptor.

In one embodiment the method of the present invention is characterized in that the modification of the base compound is replacement of one or more hydrogen atoms with one or more atoms of greater electronegativity compared to hydrogen, preferably with a halogen atom, more preferably with a fluorine atom.

In one embodiment the method of the present invention is characterized in that the evaluation of binding of the modified or base compound in the binding pocket model is carried out using steric and/or energetic criteria measured and/or determined during simulation of binding.

In one embodiment the method of the present invention is characterized in that the modified compound binds and preferably activates the target opioid receptor in conditions of inflammation-associated pH in inflamed tissue, preferably at pH values between 4 and 7, such as 4, 4.5, 5, 5.5, 6 or 6.5, more preferably between 5 and 7, or any other pH value below 7.

In one embodiment the method of the present invention is characterized in that the modified compound exhibits inflammation-specific peripheral analgesic function in inflamed or injured tissues, preferably without causing central or intestinal effects.

A further aspect of the invention relates to a method as described herein, further comprising formulating the identified modified compound in a pharmaceutically acceptable form.

A further aspect of the invention relates to a method as described herein, comprising the method as described herein and furthermore preparing the compound identified with a pharmaceutically acceptable carrier.

In one embodiment the method of the present invention is characterized in that determination of the pKa of a modified compound comprises determination of the pKa for each conformation of said modified compound and calculation of the pKa based on a weighted average of the pKa for each conformation, whereby the weighted average corresponds to the statistical weight for each conformation.

The pKa value of any given modified compound can be determined by various methods known to those skilled in the art, either computationally, or for example via chemical experimentation involving titration.

In one embodiment the method of the present invention is characterized in that simulation of binding is carried out using multiple 3D conformations of the modified compound, whereby determination of 3D conformation of a candidate compound comprises determination of potential 3D conformations of a candidate compound as meta-stable conformations in position space and determination of the statistical weights for each conformation.

In one embodiment the method of the present invention is characterized in that simulation of binding is carried out using binding path analysis.

In one embodiment the method of the present invention is characterized in that the 3D binding pocket model is constructed by determination of size of the atoms of the binding pocket, charge of the atoms of the binding pocket and preferably charge of regions of the binding pocket model.

In one embodiment the method of the present invention is characterized in that the binding pocket model is constructed using homology modelling.

In one embodiment the method of the present invention is characterized in that the binding pocket model is constructed using amino acid sequences and/or crystal structures of the mu-opioid receptor, delta-opioid receptor, kappa-opioid receptor, rhodopsin, opsin, human adrenergic receptor, human adenosine receptor and/or the beta-androgen receptor.

In one embodiment the method of the present invention is characterized in that the steric criteria for evaluation of binding of the modified or base compound in the binding pocket model are determined by measurement of the distance between reference atoms of the modified compound and the binding pocket model.

In one embodiment the method of the present invention is characterized in that the reference atom of the modified compound is the nitrogen atom that can be protonated or non-protonated.

In one embodiment the method of the present invention is characterized in that the reference atom of the binding pocket model is the aspartic acid residue 147 (Asp147) of the mu-opioid receptor.

In one embodiment the method of the present invention is characterized in that the energetic criteria for evaluation of binding of the modified or base compound in the binding pocket model are determined by calculation of the Leonard Jones potential and/or Coulomb interactions.

In one embodiment the method of the present invention is characterized in that calculation of the energetic binding criteria for the modified and/or base compound is carried out for binding of the compound in the binding pocket model, and then compared to binding of the compound in water as a reference value.

In one embodiment the method of the present invention is characterized in that the in vitro validation comprises of measurement of GTPγS binding, G-protein activation, cAMP accumulation, modulation of TRVP1 currents, modulation of stimulus-evoked action potentials and/or any other suitable in vitro assay for determining opioid receptor activation by tested compounds.

In one embodiment the method of the present invention is characterized in that the in vitro validation comprises use of embryonic kidney cells, preferably HEK 293 cells, sensory neurons and/or cultured dorsal root ganglion (DRG) neurons.

In one embodiment the method of the present invention is characterized in that the in vivo validation comprises the CFA-induced hindpaw inflammation assay, in vitro skin-nerve preparations and/or any other suitable in vivo assay for determining analgesic effects of the tested compounds.

### DETAILED DESCRIPTION OF THE INVENTION

A method for identifying candidate compounds that bind and preferably activate opioid receptors in a pH-dependent manner is provided herein. Candidate compounds identified via the claimed method are preferably tested in transfected cell lines, cultured sensory neurons, in the *in vitro* skin-nerve preparation, in knock-in mice carrying mutant opioid receptors and/or in animal models of inflammatory pain.

The compounds identified and the method of the present invention transcends traditional concepts and methods of pain treatment by focusing on the specific activation of peripheral opioid receptors in injured tissues. The agonists identified via the method described herein have been demonstrated to exhibit analgesic activity in animal models of inflammatory pain *in vivo.* The present invention therefore relates to the influence of an inflamed (acidic) environment on opioid receptor-ligand interactions and provides a method for identifying candidate compounds that selectively bind and preferably activate a target opioid receptor in a pH-dependent manner in injured tissue.

More specifically, the method in a preferred embodiment relates to (i) simulating the conformations of opioid receptors, their binding pocket (and their G-protein binding domain) at different pH values and protonation states; (ii) analyzing conformational changes of opioid agonists at different pH values; (iii) using transfected human embryonic kidney (HEK-293) cells, cultured primary afferent neurons and *in vitro* skin-nerve preparations to examine opioid agonist binding, GTPγS binding, cAMP accumulation and modulation of TRPV1 currents and action potentials by opioid agonists at varying pH values; (iv) analyzing receptor docking of protonated versus deprotonated opioid agonists; (v) using this information to design novel opioid agonists that selectively activate opioid receptors at acidic but not at normal pH values; and (vi) testing these compounds for antinociceptive efficacy animal models of inflammatory pain.

The identification method of the present invention applies computational methods and software for the simulation of binding processes and conformational stability of the opioid receptor and opioid ligands at different pH-values, and for the computational design of novel opioid agonists.

The inventors have developed computational methods and software for a stable and efficient analysis of conformational changes of molecular systems ("conformation dynamics"). These methods allow determination of the main conformations of a molecule with their thermodynamical weights and transition probabilities. In this context, conformations are defined as almost invariable (metastable) subsets in the position space, not as local minima of the energy landscape of a molecule. Conformation dynamics is the ideal method to generate a canonical ensemble simulation in a well-conditioned way including error analysis. For the simulation of ligand-receptor binding via conformation dynamics, much of the protein is held fixed, whereas the active binding site and the ligand are fully sampled. The simulation of the path of a ligand into the binding pocket of a receptor (and preferably of downstream conformational alterations influencing G-protein binding) has been developed. Binding affinities can be estimated and, thus, rational drug development is feasible. Whereas standard computer methods such as docking routines focus on the enthalpic contributions to binding affinity, conformation dynamics simulations include entropical effects of the binding process. Entropical effects are particularly important for the pH dependency of opioid receptor-ligand interactions and consequent receptor function.

The fentanyl derivatives identified by the method of the present invention function as opioid receptor agonists, which bind and activate target opioid receptors in a pH-dependent manner. The fentanyl compounds as described herein relate to examples of compounds identified by the method of the present invention and limit in no way the method itself. Other compounds with different chemical structures can be identified using the method described herein. The fluoro-substituents at positions R1, R2, R3, R4 of the herein described fluoro-fentanyl derivatives exhibit an enhanced electronegativity when compared to hydrogen and therefore lead to an enhanced electronegativity of the fentanyl derivative molecule, thus providing the compounds with their pH-dependent receptor agonist activity. Through the fluor-modification of the fentanyl backbone the overall conformation or three-dimensional structure of the molecules remains unchanged, or only changed to a minor extent, whereas the electronegativity is significantly reduced.

The "pH-dependent binding" between the compounds as described herein and the opioid receptor relates to an enhanced binding at pH values less than 7, in comparison to values above 7. In a preferred embodiment the binding between compound and receptor as well receptor activation is enhanced proportionally with lowering pH values. Obviously at very low pH values the receptor protein may undergo significant conformational change and/or denaturation, so that the binding and activation no longer occurs in an improved manner. Therefore the binding between F-fentanyl and receptor as well as receptor activation occurs preferably at pH values of 4, 4.5, 5, 5.5, 6 or 6.5, or any other pH value below 7, whereby the binding and activation occurs most preferentially at pH values between 5 and 6. Although some binding may occur at either higher or lower pH values as explicitly provided herein, the binding and activation of the receptor (and associated pain-relief in vivo) occurs most preferentially at pH levels, which are naturally lowered to approximately 4, 4.5, 5, 5.5, 6 or 6.5, or any other pH value below 7, after injury or inflammation of tissue.

The activation of the receptor occurs in a preferred embodiment via binding of the agonist to the receptor. However, the binding of the agonist to the receptor may be either a stable or transient event, of either strong or weak interaction, such that the receptor is activated preferably by agonist-receptor physical interaction.

The binding of compounds identified using the method provided herein to the opioid receptors and the subsequent receptor activation as described herein, relates preferably to compounds according to formula I and the mu-opioid receptor. However, due to structural and/or functional similarities between various opioid receptors and/or signalling pathways involved in analgesic effects, the binding and receptor activation by the claimed compounds may also occur with opioid receptors in addition to the mu-receptor, such as the delta-receptor, kappa-receptor, or other related opioid receptors.

The terms "inflammatory pain" and "inflammation-associated pain" are used interchangeably. Said inflammation-associated pain relates to any kind of pain experienced by a subject when inflammation is involved. The compounds of the present invention are intended for use in the treatment of arthritis, skin inflammation, inflammatory back pain, headache (including neurogenic migraine), inflammatory lesions of the nervous system (neuropathic pain), cancer pain, disorders of the immune system (HIV/AIDS, multiple sclerosis), traumatic pain, postoperative pain, inflamed joints, dental surgery, visceral pain, bone pain, burn injury, and/or eye lesions. These disorders are intended as examples of conditions that are associated with inflammatory pain and are not intended as a limiting disclosure.

The compounds detected by the method of the present invention therefore exhibit the surprising advantage that unacceptable side effects of traditional analgesic treatments are avoided, such as the central actions of opioids (e.g. sedation, respiratory depression, nausea, addiction, tolerance), the intestinal effects of opioids (constipation, ileus), the gastrointestinal and cardiovascular effects of cyclooxygenase (COX) inhibitors (e.g. bleeding, ulcers, thrombo-embolic complications) and the adverse effects of anticonvulsants and antidepressants (e.g. sedation, ataxia, arrhythmias, coronary vasoconstriction).

### FIGURES

The invention is further described by the figures. These are not intended to limit the scope of the invention.

### Brief description of the figures

- Figure 1.: Two different sub-conformations (SC) of protonated morphine
- Figure 2.: Morphine with indexed hydrogens
- Figure 3.: Competitive binding measuring the affinity of control mu-agonists to the mu-receptor
- Figure 4.: Suppression of cAMP-accumulation by F-fentanyl
- Figure 5.: Antinociceptive effects of opioid ligands in vivo after intraplantar (i.pl.) injection into the hindpaw of rats
- Figure 6.: Antinociceptive effects of opioid ligands in vivo after intravenous injection

### Detailed description of the figures

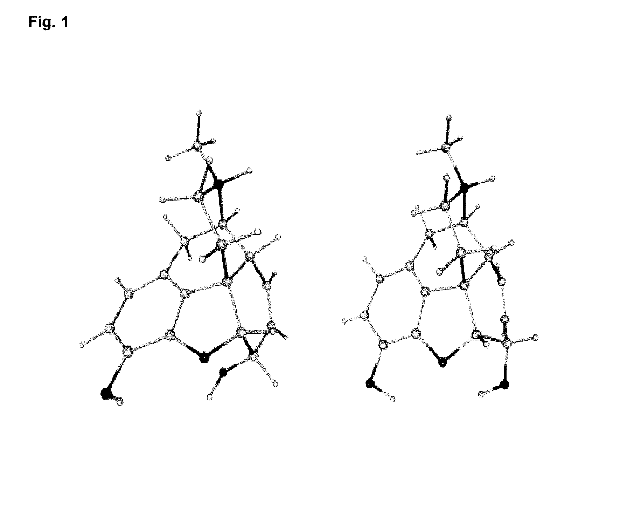

Figure 1: Two different conformations (or sub-conformations (SC)) of protonated morphine: SC1 (left) and SC2 (right) have different positions of 6-OH. Transition rates between SC1 and SC2 for protonated morphine (N+) and for deprotonated morphine (N) are shown in Tab. 1 and Tab. 2.

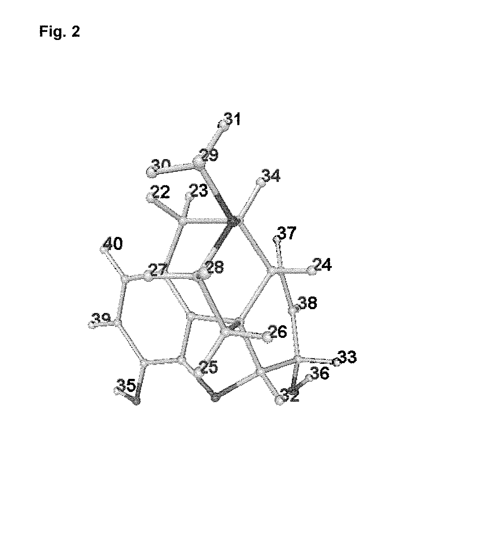

Figure 2: Morphine with indexed hydrogens. Hydrogens were replaced by fluorine and corresponding pKa-values calculated. Only results with decreased pKa-values are shown.

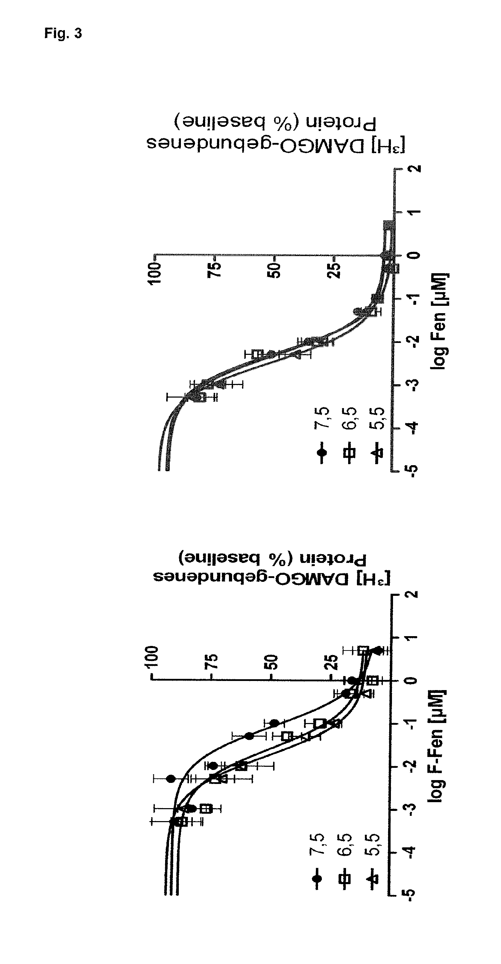

Figure 3. Competitive binding experiments measuring the affinity of control muagonsists to the mu-receptor.

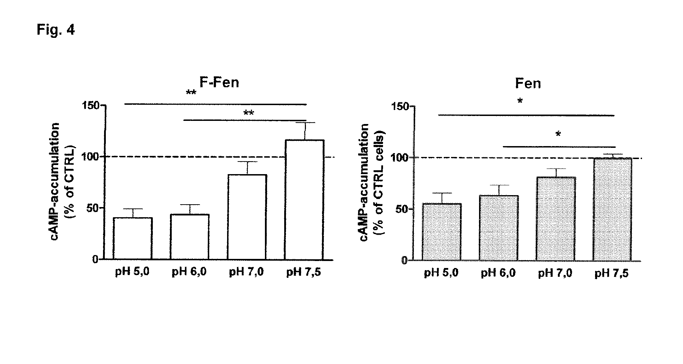

Figure 4. Suppression of FSK/IBMX-stimulated cAMP-accumulation by F-fentanyl (white) and fentanyl (grey).

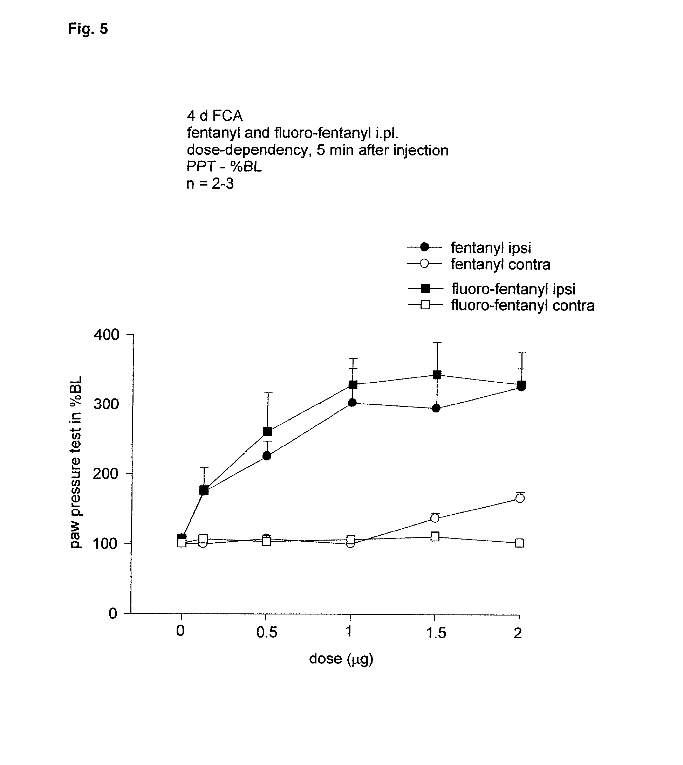

Figure 5. Antinociceptive effects of opioid ligands in vivo. Antinociceptive effects (PPT elevations in % baseline) in inflamed (ipsi) and noninflamed (contra) paws following i.pl. (intraplantar) injection of drugs in rats.

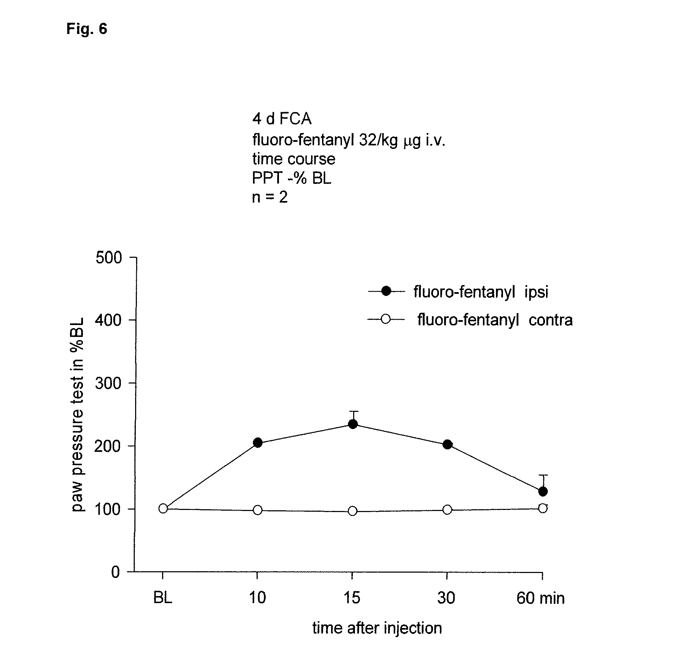

Figure 6. Antinociceptive effects of opioid ligands in vivo. Antinociceptive effects (PPT elevations in % baseline) in inflamed (ipsi) and noninflamed (contra) paws following i.v. (intravenous) injection of drugs.

### EXAMPLES

The invention is further described by the following examples. These are not intended to limit the scope of the invention. The experimental examples relate to identification and validation of candidate compounds using the method as described herein. The experiments are used to demonstrate the invention by way of example.

### Example 1: Analysis of mu-opioid receptor conformations

Since an x-ray crystal structure of the mu-opioid-receptor was not available, the crystal structure of rhodopsin served as the basis for the modeling of the mu-opioid receptor. An accurate 3D structure model of the rat mu-opioid receptor from a protein database (PDB, 2iql, 2iqo) (Fowler et al. Biochemistry 43, 15796-810, 2004) was used. The human and rat mu-receptor share 95 % amino acid identity. The Fab fragment of the anti-morphine antibody 9B1 was also used, which exists as a crystal structure with a bound morphine molecule (PDB-Id 1Q0Y). The binding pockets have similar amino acid compositions: In 9B1, morphine's protonated nitrogen forms a salt bridge with GluH50, in the mu-receptor with Asp147.

### Example 2: Conformational space of the morphine molecule at different pH values

Using software specifically designed for the purpose (ZIBgridfree, Weber, M., Scharkoi, O., FU Berlin, 2006, 2007) it was found that both protonated and deprotonated morphine have one main metastable conformation with two subconformations at 300 K (Fig. 1, Tab. 1 and Tab. 2). For protonated morphine the statistical weights are 51.85 % for subconformation 1 and 48.04 % for subconformation 2. For deprotonated morphine the statistical weights are 24.49 % for subconformation 1 and 75.50 % for subconformation 2.

| Tab. 1. Transition rates between SC1 and SC2 (N-protonated) | | |
|---|---|---|
| N+ | 1 | 2 |
| 1 | 0.55 | 0.45 |
| 2 | 0.46 | 0.54 |
| | | |
| Tab. 2. Transition rates between SC1 and SC2 (N-unprotonated) | | |
| N | 1 | 2 |
| 1 | 0.265 | 0.735 |
| 2 | 0.243 | 0.757 |

### Example 3: Design of new ligands by modification of agonist pKa values

To be activated, the mu-opioid receptor preferably requires an opioid agonist with protonated N-group (Li et al, Life Sciences, vol. 65, 2, 175-185, 1999). The pKa value of the N-group of most opioids is about 8 (e.g. 8.2 for morphine, 8.4 for fentanyl). Thus, the N-group is protonated both in inflamed (pH 5-6) and in healthy (pH 7.4) tissue and opioid receptors are activated in both environments. If the pKa value of an agonist's N-group could be decreased to about 6, it would be protonated in inflamed but deprotonated in normal tissue and, therefore, it would be active only in inflamed tissue. One possibility to do this is replacement of hydrogen by fluorine atoms, which should have no major influence on the dynamic behavior of the opioid agonist. Fluorine is very electronegative and hence attracts protons and decreases the pKa-value.

The following modifications were examined by computational modeling: (i) Systematic replacement of single, pairs or triples of hydrogen atoms of an initial opioid by fluorine atoms, calculation of the pKa-value of resulting molecules by Gaussian 09, and selection of those with values of about 6. (ii) Selection of molecules with surface, conformational space and binding characteristics most similar to the original opioid. This design concept was applied to morphine, a rigid molecule perfectly suited for sampling and illustration, yielding the results shown in Fig. 2. The conformational spaces of the new molecules (F5, F6), were then compared via ZIBgridfree. Protonated morphine, F5 and F6 have similar conformational spaces, i.e. one metastable conformation with two subconformations (Fig. 1) and similar statistical weights (Tab. 4). The electrostatic and geometrical properties of the surfaces of morphine and the modified molecules were compared via surface matching which yielded a higher score for F5 (Tab. 5).

| Tab. 3. Respective hydrogens replaced by fluorine and corresponding pKa-values. | | |
|---|---|---|
| Structure | Hydrogen Index | pKa-value |
| Morphine | --- | 8.66 |
| F1 | 29 | 4.36 |
| F2 | 30 | 3.14 |
| F3 | 31 | 3.93 |
| F4 | 27 | 3.83 |
| F5 | 28 | 6.20 |
| F6 | 34 | 6.15 |

| Tab. 4. Statistical weights of sub-conformations 1 and 2 | | |
|---|---|---|
| | 1 | 2 |
| morphine | 0.52 | 0.48 |
| F5 | 0.65 | 0.35 |
| F6 | 0.57 | 0.43 |
| | | |

| Tab. 5. Surface properties | | | |
|---|---|---|---|
| Structure | morphine | F5 | F6 |
| Score | 1 | 0.49 | 0.47 |

### Example 4: Analysis of receptor docking

The binding behavior of protonated F5 and F6 was compared to that of morphine. Protonated F5 seemed to have the same stable position in the binding pocket of the mu-receptor as protonated morphine. The positions of protonated F6 and deprotonated morphine were less stable (Fig. 3). We performed a similar analysis using the Fab fragment of the anti-morphine antibody 9B1 (PDB-Id 1Q0Y) (see goal i). Replacement of one hydrogen atom of morphine by a fluorine atom did not influence its behavior in the binding pocket of 9B1 (Fig. 4).

On the basis of our simulations, F5 qualifies as a candidate compound for a drug candidate. It has a predicted pKa-value of its N-group of 6.20. The conformational space and binding behavior of protonated F5 is very similar to that of protonated morphine.

The analogous procedure was carried out for the mu-agonist fentanyl. Through this approach fluorofentanyl (F-fentanyl) was developed and was selected using the method of the present invention as a candidate compound for a drug. F-Fentanyl was subsequently synthesized and its analgesic function validated using both in vitro and in vivo approaches.

### Example 5: Mu-opioid receptor function in vitro:

We used transfected HEK-293 cells and cultured dorsal root ganglion (DRG) neurons to examine mu-agonist binding, GTPγS binding, cAMP formation and modulation of TRPV1 currents by mu-agonists. Incubation of HEK-293 cells at different pH-values was initiated after the expression of mu-opioid receptors at the membrane surface was completed and stable. Affinity and number of mu-ligand (³H-DAMGO) binding sites were slightly (nonsignificantly) lower at pH 5.5 compared to pH 7.5 (Tab. 6). Preincubation with the adenylyl cyclase activator forskolin (FSK; 10 µM) and the phosphodiesterase inhibitor 3-isobotyl-1-methylxanthin (IBMX; 2 mM) (control groups representing 100 % cAMP accumulation compared to unstimulated cells) yielded no significant differences between pH values of 5.0, 6.0, 7.0 and 7.5 (Fig. 5). However, suppression of FSK/IBMX-stimulated cAMP formation by DAMGO and morphine was markedly stronger at pH 5.0 than at pH 7.5 (Tab. 6), indicating a higher efficacy of mu-agonistinduced inhibition of adenylyl cyclase activity in acidic conditions.

| Tab. 6. Wild-type mu-receptors: mu-agonist binding and cAMP inhibition. | | | | | |
|---|---|---|---|---|---|
| | ³H-DAMGO | ³H-DAMGO | | 10 µM DAMGO | 10 µM Morphine |
| pH | K_{d} (nM) | Bₘₐₓ (fmol/mg) | pH | cAMP (% baseline) | |
| 5,5 | 5,561 | 14,04 | 5,0 | 43 ± 6 | 46 ± 11 |
| 6,5 | 2,101 | 15,50 | 6,5 | 68 ± 9 | 64 ± 13 |
| 7,5 | 1,149 | 23,95 | 7,5 | 82 ± 7 | 80 ± 10 |

Using fluoro-fentanyl (F-fentanyl) or fentanyl as mu-opioid receptor ligands the following results were obtained: Competitive binding experiments were performed using a fixed concentration of ³H-DAMGO (4 nM) in the presence of increasing concentrations (10⁻⁶ - 1 µM) of unlabelled F-fentanyl and the half maximal inhibitory concentration (IC₅₀) was calculated. The resulting IC₅₀ values were 1.03 nM (pH 5.5), 4.78 nM (pH 6.5) and 139.7 nM (pH 7.5), indicating that F-fentanyl displayed increasing affinity to mu-receptors with decreasing pH values. Thus, the lower the IC₅₀, the higher is the affinity of F-fentanyl to the mu-receptor (see also Fig. 3).

Both F-fentanyl and fentanyl produced significantly stronger reductions of FSK/IBMX-stimulated cAMP at pH 5.0 and 6.0 compared to pH 7.5 (Fig. 6). The difference between cAMP values at pH 5.0 and pH 7.5 seemed to be more pronounced for F-fentanyl than for fentanyl (F-fentanyl: **p=0.0011; fentanyl: *p=0.0118; ANOVA; Fig. 6). This demonstrates that F-fentanyl activates mu-opioid receptors predominantly during acidic conditions and is more effective in binding and receptor activation than fentanyl.

We also assessed G-protein activation following mu-receptor activation by 35S-GTPyS-binding. F-fentanyl yielded half maximal effective concentrations (EC50) of 0.25 nM (pH 5.5) and 4.14 nM (pH 7.5). The corresponding values for fentanyl were 53.37 nM (pH 5.5) and 41.91 nM (pH 7.5). Thus, F-fentanyl was functionally more potent at pH 5.5 than at pH 7.5, and it was more potent than fentanyl at all pH values.

### Example 6: Antinociceptive effects of opioid ligands in vivo

In rats with unilateral hindpaw inflammation induced by intraplantar (i.pl.) Freund's complete adjuvant, fentanyl and F-fentanyl were injected i.pl. into the inflamed paw. At doses up to 1 µg, both drugs produced dose-dependent paw pressure threshold (PPT) elevations (antinociceptive effects) in the inflamed but not in the contralateral noninflamed paw. The effects of F-fentanyl were slightly stronger (Fig. 7). Consistent with our previous studies, this indicates that both drugs activate peripheral opioid receptors in inflamed but not in noninflamed tissue (Fig. 7). At higher doses fentanyl produced effects also on the contralateral paw, suggesting that the drug was absorbed into the circulation and produced central antinociceptive effects (Fig. 7). This was not the case for F-fentanyl (Fig. 7), indicating that, even if the drug reached the CNS after absorption into the circulation, it did not activate central opioid receptors. The intravenous (i.v.) injection of high doses (32 µg/kg) was lethal in the case of fentanyl (due to central respiratory depression). The same i.v. dose of F-fentanyl did not elicit any noticeable central effects but produced selective antinociception in the inflamed paw (Fig. 8). This indicates that peripheral opioid receptors in the injured tissue but no central receptors were activated.

### Methods used in the examples of the present invention

Details regarding software and computer-based methods are provided in the examples of the present invention

Cell culture and transfection: HEK293 cells are maintained in DMEM media supplemented with 10% fetal bovine serum and 1% streptomycin-penicillin, in 5 % CO2 at 37 °C. Cells are passaged every 2-4 days and are not used above passage number 30. HEK293 cells are plated on poly-L-lysine-coated 96 well plates for ELISA and on 100 mm diameter plastic culture dishes for binding studies. After 2 days HEK293 cells are transiently transfected with 12 µg (binding experiments) and 0.1 µg (ELISA experiments) cDNAs of rat wildtype or mutant mu-opioid receptor using FuGENE 6 Transfection Reagent (Roche Diagnostics). Twenty four h after transfection, cells are preincubated at room temperature for 20 min at varying pH values (5.5-7.4) and then processed for further experiments.

Cyclic AMP enzyme linked immunosorbent assay (ELISA): Transfected cells are cultivated in a 96-well-plate and incubated with 10 µM forskolin, 2 mM 3-Isobutyl-1-methyl-Xanthin (IBMX) in the absence or presence of morphine/DAMGO under different pH conditions (5.0; 5.5; 6.0; 6.5; 7.0; 7.4) for min 20 min at 37° C. cAMP measurements are performed using the cAMP Biotrak Enzymeimmunoassay System (Amersham Biosciences) protocol following the manufacturer's instructions. Non-bound cAMP peroxidase oxidizes tetramethylbenzidine to a blue derivate. The reaction is stopped by applying sulphuric acid resulting in the accumulation of a yellow dye. The intensity of the color is detected with an ELISA-photometer at 450 nm.

Ligand binding: Membranes of HEK 293 cells expressing mu-opioid receptors are washed twice with 10 ml of Tris buffer (Trizma Preset Crystals pH 7.4; Sigma), harvested with a scraper, homogenized and centrifuged at 42000 g and 4°C for 20 min. The pellet is homogenized in 10 ml Tris and centrifuged at 42000g and 4°C for 20 min. Protein concentration is determined using the Bradford method. Appropriate concentrations of cell membranes (200 µg) are incubated in a final volume of 400 µl Tris buffer (pH 5.5, 6.5 or 7.4) with increasing concentrations of e.g. 3H-DAMGO (0.5 nM - 16 nM) (51Ci/mmol; Amersham) (or other ligands) in the absence and presence of 10 µM unlabelled naloxone. The presence of naloxone defines non-specific binding, which typically represents 15 to 35 % of total binding. Filters are soaked in 0.1 % (w/v) polyethyleneimine solution for 15 min before using. Bound and free ligand are separated by rapid filtration under vacuum through Whatman GF/B glass fiber filters, followed by four washes with cold Tris buffer. Bound radioactivity is determined by liquid scintillation spectrophotometry at 69% counting efficiency for 3H after overnight extraction of the filters in 3 ml of scintillation fluid.

Guanosine-5'-O-(3-³⁵S-thio)-triphosphate (³⁵S-GTPyS) binding: After preincubation for 20 min at varying pH values (5.5-7.4), HEK 293 cells expressing wild-type or mutant opioid receptors are washed two times with 10 ml PBS, harvested with a scraper in 10 ml ³⁵S-GTPyS assay buffer (50 mM Tris-HCL, 5 mM MgCl₂, 0.2 mM EGTA, 100 mM NaCl, and 1 mM dithiothreitol), homogenized and centrifuged at 42000 g and 4°C for 10 min. Cell pellets are resuspended in 10 ml ³⁵S-GTPyS assay buffer, homogenized and centrifuged again. Protein concentration is measured using the Bradford method. Adequate concentrations of protein (10-50 µg), varying concentrations of DAMGO (10¹² - 10 ⁻⁴ M) (or other ligands), 50 µM GDP and 0.05 nM ³⁵S-GTPyS in a total volume of 800 µl are incubated for 2 h to generate concentration-effect curves. Basal binding is detected in the absence of agonist and non-specific binding in the presence of 10 µM cold GTPyS. Bound and free ³⁵S-GTPyS are separated by vacuum filtration through GF/B filters. Quantification of bound ³⁵S-GTPyS is achieved by a liquid scintillation counter. Similar to our previous studies, saturation analysis of agonist-stimulated GTPyS binding is performed to determine the K_{d} of GTPyS and the total amount (Bₘₐₓ) of G-protein binding to the opioid receptor. Similar experiments are performed in DRG membranes obtained from the inflamed or noninflamed limbs of animals treated with intraplantar CFA, as commonly carried out in the art.

## Claims

1. Method for identifying modified compounds that bind and preferably activate a target opioid receptor in a pH-dependent manner, comprising
a) Modification of base compound to produce a modified compound,
b) Determination of the acid dissociation constant (pKa) of said modified compound,
c) Selection of said modified compound for further processing when pKa of said modified compound is less than 7, preferably between 4 and 7, more preferably between 5 and 7, and
d) Testing the binding of the modified compound in, and preferably activation of, said target opioid receptor

2. Method according to claim 1, **characterized in that** step d) is carried out by simulation of binding of the modified compound in a three-dimensional (3D) binding pocket model for said target opioid receptor.

3. Method according to the preceding claim, **characterized in that** step d) is carried out with the modified compound and base compound, successively, in any order, whereby binding of the modified compound in the binding pocket model is compared to binding of the base compound in the binding pocket model, whereby desired modified compounds are identified when the binding of the modified compound is the same or similar in comparison to the base compound.

4. Method according to any one of the preceding claims, **characterised in that** one or more of the steps a) to d) are simulations carried out by one or more computer programmes, preferably as individual software modules combined to be automatically carried out, on a computing device.

5. Method according to any one of the preceding claims, **characterised in that** the identified modified and/or base compound is chemically synthesized.

6. Method according to any one of the preceding claims, **characterized in that** the chemically synthesized modified compound and/or base compound is tested for opioid receptor binding, and preferably opioid receptor agonist function, using in vitro and/or in vivo validation methods.

7. Method according to any one of the preceding claims, **characterized in that** the base compound is an opioid or opioid derivative or analogue, which exhibits a nitrogen atom that can be protonated or non-protonated.

8. Method according to the preceding claim, **characterised in that** protonation of said nitrogen atom occurs in modified compounds with a pKa of less than 7 when at pH values less than 7.

9. Method according to any one of the preceding claims, **characterised in that** the target opioid receptor is selected from the group consisting of the mu-receptor, delta-receptor and/or kappa-receptor.

10. Method according to any one of the preceding claims, **characterised in that** the modification of the base compound is replacement of one or more hydrogen atoms with one or more atoms of greater electronegativity compared to hydrogen, preferably with a halogen atom, more preferably with a fluorine atom.

11. Method according to any one of the preceding claims, **characterised in that** the evaluation of binding of the modified or base compound in the binding pocket model is carried out using steric and/or energetic criteria measured and/or determined during simulation of binding.

12. Method according to any one of the preceding claims, **characterized in that** the modified compound binds and preferably activates the target opioid receptor in conditions of inflammation-associated pH in inflamed tissue, preferably at pH values between 4 and 7, such as 4, 4.5, 5, 5.5, 6 or 6.5, more preferably between 5 and 7, or any other pH value below 7.

13. Method according to any one of the preceding claims, **characterized in that** the modified compound exhibits inflammation-specific peripheral analgesic function in inflamed or injured tissues, preferably without causing central or intestinal effects.

14. Method according to any one of the preceding claims, further comprising formulating the identified modified compound in a pharmaceutically acceptable form.

15. A method for the production of a pharmaceutical composition comprising the method of any one of the preceding claims and furthermore preparing the compound identified with a pharmaceutically acceptable carrier.
